# EUROPEAN PATENT APPLICATION

(11) **EP 4 144 835 A1**
(43) Date of publication of application: **08.03.2023**
(21) Application number: 21797636.4
(22) Date of filing: 26.04.2021
(51) Int. Cl.: C12N 5/077, A61K 35/32, A61L 27/36, A61L 27/38, A61L 27/40, A61L 27/58, A61P 19/08, A61P 43/00

(54) **METHOD FOR CULTURING CELL POPULATION CONTAINING CARTILAGE-DERIVED TIE2-POSITIVE CELLS, AND USE OF SAID METHOD**

(30) Priority: 27.04.2020 JP 2020078504
(71) Applicant: Tokai University Educational System, Tokyo 1518677 (JP); NIPPON ZOKI PHARMACEUTICAL CO., LTD., Chuo-ku Osaka-shi Osaka 541-0046 (JP)
(72) Inventor: SAKAI, Daisuke, Isehara-shi, Kanagawa 259-1193 (JP); NAKAMURA, Yoshihiko, Isehara-shi, Kanagawa 259-1193 (JP); MATSUSHITA, Erika, Isehara-shi, Kanagawa 259-1193 (JP)
(74) Representative: Hoffmann Eitle
(86) International application number: PCT/JP2021/016563
(87) International publication number: WO 2021/220997

(57) **Abstract**

The present invention provides a means for efficiently preparing a cell population rich in functional chondrocytes that produce an extracellular matrix such as type II collagen. This culture method according to the present invention involves a method of culturing a cell population containing cartilage-derived cells positive for expression of Tie2 (cartilage-derived Tie2-positive cells), the method comprising culturing a cell population containing cartilage-derived Tie2-positive cells in a culture medium containing at least one kind of Tie2 expression enhancer other than growth factors (e.g., an extract derived from a plant of the genus *Cinnamomum).* This culturing method is preferably performed in cultureware having a culture surface coated with a coating agent (e.g., a polylysine-containing agent).

## Description

### [Technical Field]

The present invention relates to, for instance, a method of culturing a cell population containing cells that are included in cartilage and are positive for expression of a cell surface marker Tie2 (tyrosine kinase with Ig and EGF homology domain-2) (herein referred to as "cartilage-derived Tie2-positive cells" in the present specification). The present invention relates more specifically to, for instance, a method of culturing a cell population containing cartilage-derived Tie2-positive cells such that the cell population can be used in the step of inducing differentiation from cartilage-derived Tie2-positive cells into mature functional chondrocytes, for example, Col2 (type II collagen)-expressing chondrocytes.

### [Background Art]

Articular cartilage is a tissue that covers an end of a bone and absorbs an impact applied when an arm or a knee is bent. Normal articular cartilage is called "hyaline cartilage" and is characterized in that extracellular matrix (ECM) primarily composed of type II collagen and type XI collagen is formed around each cell. When hyaline cartilage is worn by aging or is damaged by injuries due to, for instance, sport or traffic accidents, the hyaline cartilage may degenerate into fibrocartilage. Once the cartilage become fibrotic, it does not return to the original state. Then, it becomes difficult to smoothly move the joint. Besides, pain and/or inflammation may occur.

Articular cartilage is a tissue lacking blood vessels, nerves, and lymphatic vessels. When cartilage damage occurs, damage healing, which will occur in other tissues, hardly works, indicating very poor self-recovering capability. In view of the above, a method of regenerating (repairing) cartilage by transplanting same species (autologous or allogenic) chondrocytes into an affected site should be performed. R&D of cell preparations containing, for instance, chondrocytes and ECM used for transplantation are in progress. Production of such a cell preparation requires a certain quantity of same species chondrocytes. For autologous transplantation, it is possible to isolate and use chondrocytes from healthy articular cartilage of each patient with, for instance, injured knee joint cartilage. However, in addition to surgery at the time of transplantation, surgery at the time of cartilage collection is also required. This damages healthy cartilage. Unfortunately, a patient's burden is thus heavy. On the other hand, for allogenic transplantation, for example, cartilage tissue may be excised by surgery from an infant patient with polydactyly and be used as a source of chondrocytes. However, the number of chondrocytes that can be collected in this way is small. Meanwhile, because the risk of viral infection is not completely negligible, for instance, it is desirable to avoid use of a mixture of chondrocytes derived from multiple patients (donors) with polydactyly in order to secure the number of chondrocytes. In both autologous transplantation and allogenic transplantation, it is important to culture a cell population containing mature chondrocytes contained in a small amount of cartilage tissue and to prepare a cell population containing a sufficient number of chondrocytes for treatment.

As described above, in order to solve the problem related to culturing and amplifying chondrocytes contained in the collected tissue, proposed is a method using pluripotent stem cells (e.g., iPS cells, ES cells) or chondrocytes obtained by inducing differentiation of somatic stem cells. For example, Patent Document 1 describes a method for producing chondrocytes from pluripotent stem cells, the method comprising the steps of: (i) culturing pluripotent stem cells under adhesion conditions in a culture medium containing one or more substances selected from the group consisting of BMP2, TGFβ, and GDF5 and an HMG-CoA reductase inhibitor; and (ii) culturing the cells obtained in step (i) under suspension conditions in a culture medium containing one or more substances selected from the group consisting of BMP2, TGFβ, and GDF5 and an HMG-CoA reductase inhibitor.

In addition, Patent Document 2 describes a spheroid for cell transplantation treatment, which spheroid is composed of a cell mixture that expresses a phenotype of cartilage-like tissue, and a method for producing the same. Examples of Patent Document 2 describe a method for producing a spheroid for cell transplantation treatment, the method comprising: subjecting chondrocytes contained in cartilage tissue or synovium-derived cells contained in synovial tissue to subculture separately in a culture dish; and further subjecting a cell mixture obtained by mixing the resulting respective subcultured cells at a predetermined ratio to shaking culture.

Meanwhile, Patent Document 3 and Non-Patent Document 1 disclose that among cells contained in an intervertebral disc tissue (nucleus pulposus (NP)), cells positive for Tie2 and/or GD2 as a cell surface marker are cells that can be called stem cells or precursor cells of NP cells; in particular, cells positive for both Tie2 and GD2 (NP stem cells in an active state) form spheroid colonies and have a potential of finally differentiating into mature NP cells through a series of differentiation cascades (in addition, have a potential of differentiating into adipocytes, osteocytes, chondrocytes, and nerve cells); and further, implantation of NP stem/precursor cells into an intervertebral disc (NP) makes it possible to produce an extracellular matrix such as type II collagen in the tissue, maintain or reconstruct the intervertebral disc tissue, and prevent or treat degenerative intervertebral discs. As a more specific embodiment, Patent Document 3 and Non-Patent Document 1 disclose that spheroid colonies were formed (together with adherent colonies) by subjecting a cell population contained in an intervertebral disc tissue (NP) to suspension culture in a methylcellulose medium; such spheroid colonies are derived from the Tie2-positive (and GD2-positive) cells described above; and type II collagen and proteoglycan are expressed in spheroid colonies (some cells thereof) (see, for example, Examples, paragraphs 0067 and 0070, and others of Patent Document 3).

However, Patent Documents 1 to 3 and Non-Patent Document 1 neither describe nor suggest that Tie2-positive cells are contained in cartilage and that Tie2-positive cells can be efficiently amplified or differentiated into chondrocytes under specific conditions.

Note that Patent Document 3 and Non-Patent Document 1 disclose that in order to maintain Tie2-positive NP cells (disc NP stem/precursor cells), a signaling mechanism between Tie2 (a receptor) and Ang-1 (Angiopoietin-1, a ligand) is required; and Tie2-positive cells can be amplified by culturing in the presence of Ang-1 (co-culturing with AHESS 5 strongly expressing Ang-1), and Ang-1 is thus considered to be a niche factor that controls the differentiation hierarchy of NP cells (Examples: paragraphs 0049, 0069, 0075, and others).

By the way, Tie2 is also expressed in vascular endothelial cells, and it is known that when Tie2 is activated, maturation, normalization, or stabilization of blood vessels is brought about, for example, disorganized blood vessel amplification (angiogenesis) observed in, for instance, tumors, rheumatoid arthritis, diabetic retinopathy, hyperlipidemia, or hypertension can be suppressed, and wrinkles can be prevented and improved. Examples of the Tie2 activator having such an action include an extract-derived from a plant of the genus *Cinnamomum* (what is called cinnamon powder, Patent Document 4), olive fruit extract (Patent Document 5), as well as quillaia, *Engelhardia roxburghiana*, ginkgo, oyster, turmeric, chrysanthemum, jujube, Chinese matrimony vine, camomile, butcher bloom, hawthorn, star fruit, pink porcelain lily, lotus, rooibos, Indian date, Chinese quince, guajava, long pepper, Siberian ginseng, mango ginger, Chinese ginseng, autumn olive, saltwort, *Kalopanax pictus*, Japanese clethra, *Hemerocallis fulva var. kwanso, Colocasia gigantea,* bladdernut, harlequin glory bower, *Stauntonia hexaphylla, Pellionia minima, Quercus serrata,* sawtooth oak, Indian lettuce, star apple, psyllium, wild rocambole, bayberry, *Gleditsia officinalis Hemsl., Polygonatum rhizome, Polygonatum odoratum,* trichosanthes seed, or Morinda officinalis root (Patent Documents 6 to 10). Various animal/plant-derived extracts have been disclosed. Further, examples of a proposed component that brings about the Tie2 activation action include ursolic acid, colosolic acid, 3-O-galloylprocyanidin B-1, linolenic acid, 13-hydroxy-9Z,11E,15E-octadecatrienoic acid, procyanidin B-2, epicatechin-(4β-6)-epicatechin(4β-8)-epicatechin, procyanidin C-1, astragaloside VIII, soya saponin I, 3'-O-methyl gallocatechin, pipernonaline, syringaresinol, 2-methoxycinnamaldehyde, eleutheroside E, eleutheroside E1, sesamin, eudesmin, sylvatesmin, pinoresinol, yangambin, forsythinol, or coumarin (Patent Documents 7 and 12 to 14).

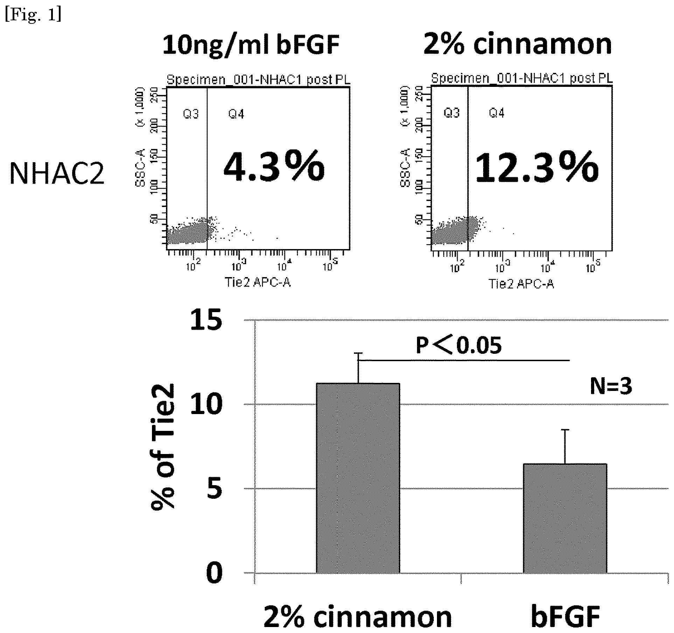

For example, Patent Document 4 provides an experiment (Examples) about the "Tie2 activation agent" such that when "hemocyte Baf3 cells strongly expressing Tie2" or "normal human umbilical vein endothelial cells (HUVEC)" were cultured in a medium containing a cinnamon twig hot water extract, the Tie2 protein expressed in these cells was found to be more phosphorylated than that in the control by Western blotting (e.g., paragraphs 0024 to 0027, Figs. 1 to 3).

However, Patent Document 4 to 14 neither describe nor suggest use of the Tie2 activator in the culture of Tie2-positive cells included in a cell population obtained from cartilage, except for blood cells (hematpcytes) and/or vascular endothelial cells, or what kinds of effects are exerted thereby.

### [Prior Art Documents]

### [Patent Documents]

Patent Document 1: WO 2016/133208
Patent Document 2: Japanese Patent Laid-Open No. 2011-41472
Patent Document 3: Japanese Patent No. 5863639 (corresponding to WO 2011/122601)
Patent Document 4: WO 2009/123211
Patent Document 5: WO 2016/060249
Patent Document 6: WO 2012/073627
Patent Document 7: Japanese Patent Laid-Open No. 2012-236795
Patent Document 8: Japanese Patent Laid-Open No. 2011-201811
Patent Document 9: Japanese Patent Laid-Open No. 2011-102275
Patent Document 10: Japanese Patent Laid-Open No. 2011-102274
Patent Document 11: Japanese Patent Laid-Open No. 2011-102273
Patent Document 12: Japanese Patent Laid-Open No. 2014-97977
Patent Document 13: Japanese Patent Laid-Open No. 2013-241356
Patent Document 14: Japanese Patent Laid-Open No. 2011-102272

### [Non-Patent Documents]

Non-Patent Document 1: Sakai D et al., Nat Commun. 2012; 3: 1264

### [Summary of the Invention]

### [Problem to be Solved by the Invention]

In order to produce an same species (autologous or allogenic) chondrocyte-containing cell preparation used for treatment of, for instance, articular cartilage damage, it is necessary to have some sufficient amount of functional chondrocytes that produce an extracellular matrix such as type II collagen and proteoglycan. In particular, in order to enhance the therapeutic effect when the cell preparation is administered to a patient, it is important to prepare a cell population rich in functional chondrocytes that can produce a large amount of extracellular matrix such as type II collagen as efficiently as possible. Further, there is a problem that even chondrocytes collected from hyaline cartilage are likely to change into fibroblast-like cells by culture. When such fibroblast-like chondrocytes are transplanted, fibrocartilage is formed in the repaired tissue, leading to a possibility that a damaged site cannot be treated by hyaline cartilage.

The present invention addresses the problem of providing a means for efficiently preparing a cell population rich in functional chondrocytes that produce an extracellular matrix such as type II collagen.

### [Means for Solving the Problems]

The present inventors have found that cartilage also contains Tie2-positive cells that include stem/precursor cells having a potential to differentiate into chondrocytes. In view of the above, the present inventors have conducted research, focusing on the state of a cell population to be cultured and its culture conditions when a cell population containing such cartilage-derived Tie2-positive cells (cartilage stem/precursor cells) is cultured, and as a result, have discovered the following special technical features that can contribute to providing a solution to the above problems.

The method of culturing a cell population containing cartilage-derived Tie2-positive cells according to the present invention includes culturing a cell population containing cartilage-derived Tie2-positive cells in a culture medium containing at least one kind of Tie2 expression enhancer other than growth factors.

It has been known that in Tie2-positive cells like hematopoietic stem cells, Angiopoietin-1 (Ang-1), an intrinsic ligand for Tie2 (receptor tyrosine kinase), is bound to promote Tie2 activation (phosphorylation). A procedure for culturing Tie2-positive cells in an Ang-1-containing culture medium (i.e., a procedure for enhancing expression of Tie2) is a known conventional technology (e.g., in the prior art Patent Document 3). Similarly, FGF2 (bFGF) is likewise known as a growth factor having an action of enhancing the expression of Tie2, and a method of culturing Tie2-positive cells in an FGF2-containing culture medium is also known.

However, the present inventors have utilized a Tie2 expression enhancer, the kind of which is different from growth factors such as Ang-1 and FGF2, for example, a Tie2 expression enhancer that is an animal/plant-derived extract such as cinnamon powder extract, for culturing a cell population containing cartilage-derived Tie2-positive cells, which has not been reported so far. In particular, the Tie2 expression enhancer that is a plant-derived extract has been used in combination with a growth factor(s) such as FGF2. In this case, the cell population obtained by culture is rich in functional chondrocytes differentiated from cartilage-derived Tie2-positive cells. In addition, it was found that the cartilage-derived Tie2-positive stem/precursor cells themselves were also contained at a higher ratio than in the conventional method. That is, the cell population containing the cartilage-derived Tie2-positive cells may be cultured in a culture medium containing a Tie2 expression enhancer. This can unexpectedly enhance the expression of Tie2 in the cartilage-derived Tie2-positive cells, maintain juvenile Tie2 positive cells (with a proliferation potential, a potential to differentiate into chondrocytes, and a spheroid formation capability) in the cell population, promote differentiation into functional chondrocytes expressing, for instance, type II collagen (Col2), and increase the number of Col2-positive cells in the cell population. In this way, competing objectives can be achieved in good balance.

In a preferred embodiment, the method of culturing a cell population containing cartilage-derived Tie2-positive cells (cartilage stem/precursor cells) according to the present invention as described above is performed under a two-dimensional culture (plane culture) environment using cultureware having a culture surface coated with a coating agent containing an extracellular matrix or another biologically relevant molecule such as polylysine. Three-dimensional culture using a scaffold material such as a gel or a porous material, or suspension culture using, for instance, non-adherent cultureware can be further performed.

Based on each culture method described above, the present inventors have established a preferable method of preparing, from a cell population containing cartilage-derived Tie2-positive cells (e.g., cartilage stem/precursor cells), a cell population containing chondrocytes differentiated from the stem/precursor cells. That is, the method of preparing a cell population according to the present invention follows the above-mentioned method of culturing cartilage-derived Tie2-positive cells according to the present invention, and comprises at least a culture stage (differentiation culture stage) of differentiating into functional chondrocytes expressing, for instance, Col2 while enhancing the expression of Tie2 on cartilage-derived Tie2-positive cells in the cell population and keeping the cells in a juvenile state. Such a method of preparing a cell population makes it possible to eventually prepare a cell population in which the absolute number or the percentage of functional chondrocytes, particularly chondrocytes positive for a cell marker such as Col2, in the cell population is dramatically improved as compared with the conventionally known preparation method. In the differentiation culture stage as described above, even when the number or percentage of, for instance, Col2-positive cells reaches a certain level, the Tie2-positive cells do not completely disappear, and a cell population having a certain level of the number or percentage of the Tie2-positive cells can be obtained. It is believed that such a cell population contains, to a certain extent, cartilage stem/precursor cells capable of producing functional chondrocytes even after administration, thereby providing better therapeutic or prophylactic effects.

If the above-described technical idea is embodied in combination with a (preferred) embodiment(s) described later in detail, the present invention can be expressed, for example, as an invention encompassing at least the following items.
[1] A method of culturing a cell population containing cartilage-derived cells which are positive for expression of Tie2 (tyrosine kinase with Ig and EGF homology domain-2) (hereinafter referred to as "cartilage-derived Tie2-positive cells"), the method comprising:
   culturing a cell population containing cartilage-derived Tie2-positive cells in a culture medium containing at least one kind of plant-derived extract having a Tie2 expression-enhancing effect.
[2] The culture method according to item 1, wherein the plant is a plant of the genus *Cinnamomum.*
[3] The culture method according to item 1 or 2, wherein the culturing is performed in cultureware having a culture surface coated with a coating agent containing an extracellular matrix and/or a polyamino acid.
[4] The culture method according to item 3, wherein the extracellular matrix is type IV collagen and/or fibronectin.
[5] The culture method according to item 3 or 4, wherein the polyamino acid is polylysine.
[6] A method of preparing a cell population containing chondrocytes differentiated from cartilage-derived Tie2-positive cells, the method comprising:
   a culture stage (hereinafter, referred to as an "differentiation culture stage") comprising a step of performing the culture method according to any one of items 1 to 5 in order to enhance expression of Tie2 on cartilage-derived Tie2-positive cells in a cell population and induce differentiation into chondrocytes.
[7] The preparation method according to item 6, wherein the chondrocytes comprise cells positive for expression of Col2 (type II collagen).
[8] The preparation method according to item 6 or 7, wherein the differentiation culture stage further comprises a step of culturing the cell population containing the cartilage-derived Tie2-positive cells in a culture medium containing at least one growth factor selected from the group consisting of FGF (fibroblast growth factor), EGF (epidermal growth factor) andAng-1 (angiopoietin-1).
[9] The preparation method according to any one of items 6 to 8, further comprising, before the differentiation culture stage, a culturing stage of amplifying the cell population containing the cartilage-derived Tie2-positive cells (hereinafter, referred to as an "amplification culture stage").
[10] The preparation method according to any one of items 6 to 9, wherein a cell population in which the cartilage-derived Tie2-positive cells also remain is obtained through the differentiation culture stage.
[11] A cell population obtained by the culture method according to any one of items 1 to 5.
[12] A culture comprising a culture medium in the culture method according to any one of items 1 to 5 and a cell population to be subjected to the culture method, being cultured, or produced.
[13] A cell population obtained through the differentiation culture stage in the preparation method according to any one of items 6 to 10.
[14] A culture comprising a culture medium for the differentiation culture stage and a cell population to be subjected to the differentiation culture stage, being cultured, or produced, in the preparation method according to any one of items 6 to 10.
[15] A composition for cell therapy, comprising the cell population according to item 10 or 13.
[16] The composition for cell therapy according to item 15, which is for treatment or prevention of a disorder, symptom, or disease in cartilage.

### [Advantages of the Invention]

The method of culturing cartilage-derived Tie2-positive cells according to the present invention makes it possible to enhance the expression of Tie2 in the cartilage-derived Tie2-positive cells, keep the cells in a juvenile state, and promote differentiation of the cells into functional chondrocytes (positive for a predetermined cell marker such as Col2). Further, a cell population rich in functional chondrocytes can be prepared by a differentiation culture stage including the step of performing such a culture method of the present invention. The cell population obtained based on such a culture method and/or preparation method of the present invention may be used to efficiently produce a cell preparation effective for treatment or prevention of a disorder, symptom, or disease in cartilage.

According to a representative embodiment of the present invention, cartilage that can be collected only in a small amount is used to efficiently amplify and differentiate Tie2-positive cells (i.e., cartilage stem/precursor cells) contained therein. Accordingly, a cell population that should elicit a high therapeutic effect upon implantation, that is, a cell population rich in functional chondrocytes having increased production of extracellular matrix such as type II collagen (and some remaining Tie2-positive cells) can be easily and reproducibly obtained in large amounts. Conventionally, it has been difficult to produce such a suitable cell population. Since the present allows for efficient production, regeneration therapy of cartilage such as a joint by administration of (a cell preparation containing) such a cell population is dramatically facilitated. As a result, industrialization becomes realistic.

### [Brief Description of Drawings]

[Figure 1] Fig. 1 is a graph and charts showing the Tie2 positive percentage of a cell population finally (after the differentiation culture second step) obtained from commercially available knee joint chondrocytes (NHAC2) when the culture method of the present invention (2% cinnamon) was performed in the differentiation culture stage (differentiation culture first step) or when the culture method as a control (comparative example) (10 ng/ml bFGF) was performed, as measured in Example 1.

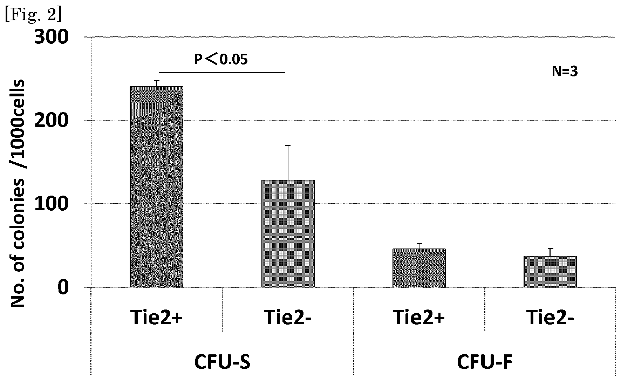

[Figure 2] Fig. 2 is a graph indicating spherical colony forming units (CFU-S) and fibroblastic colony forming units (CFU-F) after the differentiation culture second step for each of Tie2-positive cells (Tie2+) and Tie2-negative cells (Tie2-) contained in commercially available knee joint chondrocytes (NHAC2), as measured in Example 2.

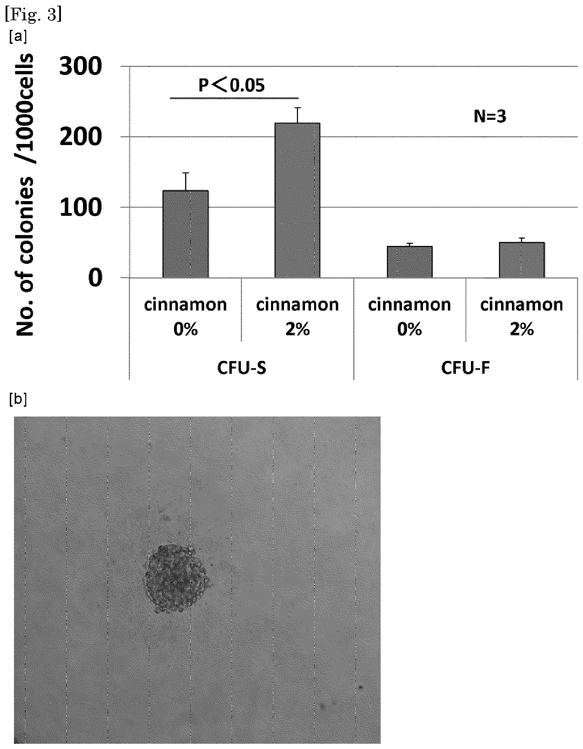

[Figure 3] Fig. 3(a) is a graph indicating spherical colony forming units (CFU-S) and fibroblastic colony forming units (CFU-F) with respect to each of a case where the differentiation culture step (2% cinnamon) according to the culture method of the present invention was performed or a case where the differentiation culture step (0% cinnamon) as a control (comparative example) was performed, in the differentiation culture stage, as measured in Example 3. Fig. 3(b) shows an optical micrograph of a spherical colony (spheroid).

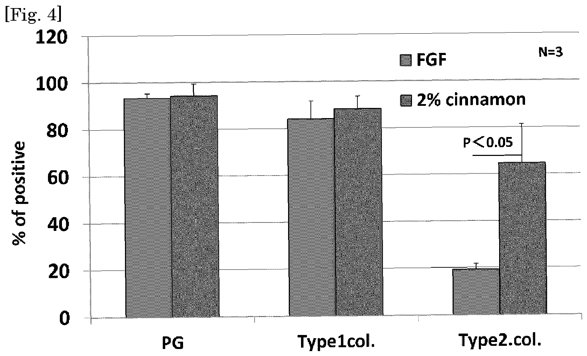

[Figure 4] Fig. 4 is a graph showing the positive rate of each of proteoglycan (PG), type I collagen (Type1 col.), and type II collagen (Type2.col.) of cell population finally obtained from commercially available knee joint chondrocytes (NHAC2) when the differentiation culture step (2% cinnamon) in the culture method of the present invention was performed or when the differentiation culture step (10 ng/ml bFGF) as a control (comparative example) was performed, in the differentiation culture stage, as measured in Example 4.

### [Mode for Carrying Out the Invention]

### -Terms-

The term "stem cell(s)" refers to a cell(s) having self-renewal capability and differentiation potential (i.e., totipotent, pluripotent, multipotent, or unipotent cells). The term "precursor cell(s)" refers to a cell(s) without self-renewal capability in a strict sense because all of the cells finally become terminally differentiated cells, but with some differentiation potential to differentiate into a predetermined cell(s) while relatively actively proliferating. (Identified) cells generally understood and called, by those skilled in the art, as "stem cells" or "precursor cells" herein correspond to "stem cells" or "precursor cells".

As used herein, the wording "stem cells and/or precursor cells" includes stem cells, precursor cells, or both, and is sometimes referred to as "stem/precursor cells". In addition, as used herein, a cell population containing stem cells and/or precursor cells may be referred to as a "stem/precursor cell population", and a cell population containing mature cells differentiated from the stem cells and/or precursor cells (i.e., terminally differentiated cells) may be referred to as a "mature cell population".

In general, the "stem cells" and the "precursor cells" are distinguishable from other cells by whether the expression of one or two or more kinds of specific genes (marker genes or cell markers) is positive or negative. That is, the "stem cells" or the "precursor cells" having self-renewal capability and/or differentiation potential as described above can also be defined as terms that refer to cells in which the expression of a specific marker gene is positive or negative, respectively.

Whether the expression of a marker gene (cell marker) is "positive" or "negative" can be determined by quantitatively or qualitatively measuring the expression level of mRNA transcribed from the gene (genome) or protein translated from the mRNA according to a common protocol. If the expression level is a certain level or higher (or higher than a certain level), the expression can be determined to be positive, and if the expression level is a certain level or lower (or lower than a certain level), the expression can be determined to be negative. The expression level of a protein can be measured quantitatively or qualitatively by an immunological assay (e.g., flow cytometry, immunostaining, or ELISA) using, for instance, an antibody or labeling agent specific to the protein. Note that the Tie2 protein is a protein expressed on the cell surface, and Col2 is a protein expressed inside a cell. Appropriate techniques (e.g., immunofluorescence staining) may be used to detect proteins present on the cell surface or inside a cell, respectively. The expression level of mRNA can be measured quantitatively or qualitatively by, for example, an assay (e.g., RT-PCR, a microarray, or a biochip) using a nucleic acid and a labeling agent or an amplification protocol (means) for nucleic acid specific (complementary) to the mRNA. The percentage (positive rate or negative rate) of cells positive or negative for expression of a given marker gene (cell marker) in a cell population can be calculated by counting the number of all cells in the cell population and the number of cells determined to be positive or negative by the above-described protocols, respectively, while using the above various techniques such as flow cytometry.

As used herein, the wording "stem cells and/or precursor cells positive for expression of Tie2", that is, "Tie2-positive cells" refers to cells where expression of Tie2 (tyrosine kinase with Ig and EGF homology domain-2) known as one of the cell markers, for example, its expression as a protein measured by flow cytometry is determined to be positive. The Tie2-positive cells in the present invention are Tie2-positive cells "derived from cartilage", that is, Tie2-positive cells present in cartilage (that can be collected from cartilage) or Tie2-positive cells obtained by subculturing the Tie2-positive cells, and are cells corresponding to (assumed to contain) "cartilage stem/precursor cells" described below.

The "chondrocytes" in the present invention refer to matured and terminally differentiated cells that account for majority of cell population in a population contained in cartilage, or cultured cells having an equivalent phenotype. The chondrocytes are preferably functional chondrocytes that are positive for at least type II collagen (Col2), among extracellular matrices, and more typically positive for proteoglycan (aggrecan), as a cellular marker. For example, cells determined to be positive for Col2 (and also positive for aggrecan) as a cell marker (preferably protein) by flow cytometry correspond to functional chondrocytes in the present invention. For extracellular matrices such as Col2 and aggrecan, the amount of each protein produced may be measured by flow cytometry, and the expression level of each mRNA may be measured by, for instance, real-time PCR as an alternative for flow cytometry. In the present invention, chondrocytes as described above, in particular, chondrocytes positive for a predetermined marker such as Col2 or aggrecan are cells obtained from cartilage-derived Tie2-positive cells by predetermined differentiation induction (differentiation culture stage) and having functionality depending on its application, that is, target cells. The chondrocytes are also known to be positive for, in addition to the above marker, CD81, CD90, CD44, CD105, CD106, and GD2, and one or two or more of them may be further used in combination with, for instance, Col2 as a cell marker(s) of chondrocytes.

As used herein, the term "cartilage stem/precursor cells" collectively refers to precursor cells (cartilage precursor cells) having at least a potential to differentiate into chondrocytes (preferably Col2-positive functional chondrocytes), stem cells (cartilage stem cells) having a self-renewal capability and a potential to differentiate into the precursor cells, which cells account for part of a cell population contained in cartilage, or cultured cells having an equivalent phenotype. Specifically, the cartilage stem/precursor cells are contained in cells positive for at least Tie2 as a marker gene. For example, cells determined to be positive for Tie2 as protein (a cell marker ) by flow cytometry correspond to (are assumed to contain) cartilage stem/precursor cells in the present invention. In one embodiment of the present invention, Tie2-positive cells can be substantially regarded as (majority of) cartilage stem/precursor cells. For example, the "Tie2-positive cells" described herein may be read as "Tie2-positive cartilage stem/precursor cells".

The "spheroid colony" in the present invention refers to a spherical cell aggregate which contains stem cells and/or precursor cells and further optionally contains cells differentiated therefrom. The "spheroid colony" is an object that may be commonly called, for instance, a "spheroid" by those skilled in the art.

As used herein, the wording "expression of Tie2 is enhanced" (enhanced expression of Tie2) means that the expression of Tie2 gene is enhanced in individual Tie2-positive cells, that is, the expression is augmented more than usual, and the expression level of mRNA or protein is increased. The wording "expression of Tie2 is enhanced" also corresponds to keeping a certain level of expression without loss of expression, that is, maintaining the expression of Tie2, in spite of being under conditions in which the expression of Tie2 gene would normally almost disappears. In addition, as a result of such enhanced expression of Tie2 in individual Tie2-positive cells, an increase in the number of cells determined to have positive expression of Tie2 mRNA or protein in the cell population (or a case where the level of expression exceeds a certain level), that is, a higher percentage of Tie2-positive cells in the cell population than usual can also be understood as an indicator of "enhanced Tie2 expression".

More specifically, for example, Tie2 protein on the cell surface is fluorescently labeled for a cell population that has undergone Tie2 expression-enhancing treatment (Tie2 expression-enhancing treatment group) or a cell population that has not undergone Tie2 expression-enhancing treatment (control group). When measured by flow cytometry, the percentage of cells determined to have a higher fluorescence intensity and more positive expression and/or an average fluorescence intensity per cell than a predetermined level may be higher in the Tie2 expression-enhancing treatment group than in the control group. In this case, it can be said that (Tie2-positive cells contained in) the cell population of the Tie2 expression-enhancing treatment group has enhanced expression of Tie2 (in other words, the Tie2 expression-enhancing treatment plays a prescribed role).

An agent that exerts the effects of "enhancing Tie2 expression" as described above is herein referred to as a "Tie2 expression enhancer" in the present invention. Note that some growth factors (e.g., FGF2) have a Tie2 expression-enhancing effect, and can be said to correspond to a kind of "Tie2 expression enhancer". Accordingly, in the case of excluding such growth factors, the agent is called a "Tie2 expression enhancer other than growth factors".

### -Culture Methods-

The method of culturing a cell population containing cartilage-derived Tie2-positive cells according to the present invention involves a method of culturing a cell population containing cartilage-derived Tie2-positive cells in a culture medium containing at least one kind of Tie2 expression enhancer other than growth factors. As used herein, the above culture method may be simply referred to as "the culture method of the present invention".

The culture method of the present invention is capable of enhancing the expression of Tie2 on cartilage-derived Tie2-positive cells and maintaining a juvenile state as a cartilage stem/precursor cell, that is, self-renewal capability and a potential to differentiate into (at least) nucleus pulposus cells. At the same time, it is possible to promote differentiation from cartilage-derived Tie2-positive cells to chondrocytes, particularly functional chondrocytes positive for expression of, for instance, Col2. In the culture method of the present invention, cartilage-derived Tie2-positive cells, the juvenile state of which is maintained, can be retained more for a longer term in the cell population (the number and/or percentage of Tie2 positive cells are increased more than those in conventional culture methods). As such, a larger amount of chondrocytes (particularly functional chondrocytes as described below) can be produced. The step of performing, so as to achieve such an objective, the culture method of the present invention at the differentiation culture stage of the preparation method of the present invention described below may be referred to as the "differentiation culture step of the present invention".

### -Preparation Method (Culturing Step)-

The method of preparing a cell population containing chondrocytes differentiated from Tie2-positive cells according to the present invention includes:
a culture stage (hereinafter, referred to as a "differentiation culture stage") comprising at least the differentiation culture step of the present invention in order to enhance expression of Tie2 on cartilage-derived Tie2-positive cells in a cell population and induce differentiation into chondrocytes. As used herein, the above preparation method may be simply referred to as the "preparation method of the present invention".

The differentiation culture stage may include a single differentiation culture step or may include a plurality of differentiation culture steps. In addition, the differentiation culture step may be a step without subculture, but may be a step optionally including one or two or more subcultures.

The differentiation culture stage is intended mainly to differentiate cartilage-derived Tie2-positive cells into chondrocytes, and in the present invention, particularly, into functional chondrocytes positive for, for instance, Col2 by culture under given conditions, and means a stage where effects therefor are exerted. That is, if the cell number and/or the percentage of chondrocytes (in particular, functional chondrocytes) are higher in the post-culture cell population than in the pre-culture cell population, the culture stage can be said to be a "differentiation culture stage".

In addition, the differentiation culture step performed at the differentiation culture stage is found to exert, in addition to the above effects, an effect of increasing or maintaining the cell number and/or percentage of cartilage-derived Tie2-positive cells, or slowing down a decrease in the cell number and/or percentage.

The differentiation culture stage may further optionally include a step other than the differentiation culture step of the present invention (a culture step under culture conditions different from those for the differentiation culture step of the present invention), which step meets the main purpose of the stage of differentiating cartilage-derived Tie2-positive cells into chondrocytes (particularly functional chondrocytes). Examples of such a step include a step of culturing a cell population containing cartilage-derived Tie2-positive cells in a culture medium free of a Tie2 expression enhancer (e.g., an animal/plant-derived extract, preferably a plant-derived extract) other than growth factors (this step is herein referred to as an "additional differentiation culture step"). Examples of the growth factor that can be added to the culture medium during the additional differentiation culture step include at least one compound selected from the group consisting of FGF, EGF, and Ang-1. The additional differentiation culture step can be performed before or after the differentiation culture step of the present invention, but is preferably performed after the differentiation culture step of the present invention. The additional differentiation culture step may be a step without subculture, but may be a step optionally including one or two or more subcultures.

The preparation method of the present invention may further optionally include a culture stage other than the differentiation culture stage. Examples of the further culture stage that can be included in the preparation method of the present invention include a culture stage for preamplifying a cell population (e.g., cartilage-derived Tie2-positive cells contained therein, chondrocytes differentiated therefrom) to be subjected to the differentiation culture stage, which culture stage is performed before the differentiation culture stage. In the present invention, the culture stage intended for such purpose is sometimes referred to as an "amplification culture stage", and the step (method) performed at the amplification culture stage is sometimes referred to as an "amplification culture step (method)".

Note that, even in the amplification culture stage (step, method), differentiation from cartilage-derived Tie2-positive cells to other cells (chondrocytes) is allowed to occur. However, the amplification culture stage is distinguished from the differentiation culture stage (step, method) of the present invention in that no Tie2 expression enhancer (e.g., an animal/plant-derived extract) other than growth factors is added to the culture medium. Meanwhile, even in the amplification culture stage (step, method), it is preferable to further optionally add a growth factor(s) such as FGF. The amplification culture step may be a step without subculture, but may be a step optionally including one or two or more subcultures.

### <Cell Population>

The cell population to be subjected to each culturing step included in the culture method of the present invention or the preparation method of the present invention (i.e., in each culture stage) (herein collectively referred to as a "pre-culture cell population") basically has a given percentage and/or number of each of cartilage-derived Tie2-positive cells or other cells, particularly (functional) chondrocytes differentiated from cartilage-derived Tie2-positive cells in the present invention. In addition, basically any ratio between stem/precursor cells and other cells or any ratio between stem cells and precursor cells included in the Tie2-positive cells of the pre-culture cell population is set. The composition of the pre-culture cell population can be adjusted, if appropriate, according to an embodiment of the present invention while the effects etc. in each culture method or each culturing step are considered.

The pre-culture cell population may be prepared or provided according to a conventional procedure. For instance, a cell population included in *in vivo* collected cartilage may be used as the pre-culture cell population. In this case, first, cartilage is finely cut (chopping) into an appropriate size while using an instrument such as a surgical curved knife. Subsequently, the resulting material is treated with a protease such as collagenase to disperse cells. The cells are, for instance, optionally filtered, centrifuged, and/or washed. These treatments enable a cell population included in the cartilage to be isolated and recovered. Cartilage may be collected from a variety of cartilage-containing tissues including: hyaline cartilage (e.g., articular (e.g., knee, elbow) cartilage, costal cartilage, tracheal cartilage, nasal septal cartilage); elastic cartilage (e.g., auricular cartilage); or fibrocartilage (e.g., meniscus). As long as the effects of the present invention are exerted, the origin of the tissue is not particularly limited. However, for example, the tissue is preferably hyaline cartilage such as articular cartilage. The cell population (cell population containing cartilage-derived Tie2-positive cells) thus obtained can be used as a pre-culture cell population for the culture method of the present invention or the step at the differentiation culture stage (e.g., the differentiation culture step of the present invention, the additional differentiation culture step optionally performed), or the step at a stage (e.g., an amplification culture stage), other than the differentiation culture stage, further optionally performed. As such, the cell population (primary cells or subcultured cells) prepared from articular cartilage (knee) or other tissues is also sold as a product (a kit optionally including a culture medium, instructions, reagents, and so on), and may also be used as a pre-culture cell population containing cartilage-derived Tie2-positive cells.in the present invention.

The cell population separated from cartilage or the cell population included in the cartilage (a cell population-containing cartilage) prepared as described above may be cryopreserved according to a conventional procedure until being subjected to the next culture method or culturing step. The cryopreserved cell population or cartilage may be thawed according to a conventional procedure when the next culture method or culturing step is started. During cryopreservation and thawing, favorable treatments for the cell population or cartilage may be combined. For example, a cryoprotectant (e.g., DMSO) may be added during cryopreservation, and in this case, it is only necessary to remove the cryoprotectant under suitable conditions during thawing.

The cell population obtained through each culturing step in the culture method of the present invention or the preparation method of the present invention (i.e., in each culture stage) (herein collectively referred to as a "post-culture cell population") basically has a given percentage and/or number of each of cartilage-derived Tie2-positive cells and other cells, particularly (functional) chondrocytes differentiated from cartilage-derived Tie2-positive cells in the present invention. In addition, basically any ratio between stem/precursor cells and other cells or any ratio between stem cells and precursor cells included in the Tie2-positive cells of the post-culture cell population is set. The composition of the post-culture cell population can be adjusted, if appropriate, according to an embodiment of the present invention while use etc. of the cell population obtained by each culture method or each culturing step is considered.

The post-culture cell population may be recovered from the culture medium according to a routine procedure and subjected to the next culture method or culturing step, or subjected to another method or step such as preparation of a cell preparation.

The cell population during each culturing step in the culture method of the present invention or the preparation method of the present invention (i.e., in each culture stage) (herein collectively referred to as an "in-culture cell population") basically has a given percentage and/or number of each of cartilage-derived Tie2-positive cells and other cells, particularly (functional) chondrocytes differentiated from cartilage-derived Tie2-positive cells in the present invention. In addition, basically any ratio between stem/precursor cells and other cells or any ratio between stem cells and precursor cells included in the Tie2-positive cells of the in-culture cell population is set. The composition of the in-culture cell population is a composition in the process of transition from the pre-culture cell population to the post-culture cell population. For example, the percentage of cartilage-derived Tie2-positive cells with respect to the in-culture cell population (herein simply referred to as the "Tie2-positive rate") is usually a value in the range (inclusive) between the Tie2-positive rate in the pre-culture cell population and the Tie2-positive rate in the post-culture cell population. However, the value is permitted to be temporarily out of the range. The composition of the in-culture cell population varies depending on an embodiment of the present invention and depending on, for instance, the number of days and the number of passages in the culture method of the present invention or each culturing step.

The "human or other animal" (donor) from which each cell population is derived can be selected in consideration of, for instance, use of the cell population finally obtained by the culture method of the present invention or the preparation method of the present invention or use of the cell population obtained by each culturing step included in the preparation method of the present invention (at each culture stage). In an exemplary embodiment of the present invention, it is possible to prepare a cell population for producing a cell preparation so as to prevent or treat, for instance, a given disease or symptom. In this case, the "human or other animal" is an organism of the same species as a subject (recipient) receiving the cell preparation, and is preferably a human. In addition, the donor and the recipient may be the same individual (autologous) or other individuals (allogenic).

### - Cell Population Involving Differentiation Culture Stage

The cell population to be subjected to the culture method of the present invention, or the cell population to be subjected to the step included in the differentiation culture stage of the present invention (e.g., the differentiation culture step of the present invention, the additional differentiation culture step optionally performed) (herein referred to as a "pre-differentiation culture cell population") is a primary or subcultured cell population derived from a human or other animal, such as a commercially available cartilage-derived subcultured cell population. For example, a cell population contained in cartilage collected from the body of a human or other animal, or a cell population (established a cell strain) obtained by subculturing such a cell population may be used as the pre-differentiation culture cell population, and such a cell population is also commercially available.

The Tie2-positive rate in the pre-differentiation culture cell population varies depending on, for instance, individual differences of collected cartilage, and is not particularly limited. However, it is preferable that the percentage and/or the cell number of cartilage-derived Tie2-positive cells should be as high as possible. For example, a cell population derived from a donor (e.g., a young individual) that has a good niche in cartilage may be such a preferred cell population.

Use of the cell population obtained by each step included in the differentiation culture stage of the present invention (herein collectively referred to as a "post-differentiation culture cell population) is not particularly limited. The composition of the post-differentiation culture cell population, for example, the Col2-positive (cartilage) cell percentage (herein referred to as a "Col2-positive rate") and Tie2-positive cell percentage (Tie2-positive rate) as well as the numbers of these cells vary depending on, for instance, the pre-differentiation culture cell population and/or individual differences of collected cartilage, but can be adjusted, if appropriate, corresponding to its application. For example, the cell population used for producing a cell preparation for transplantation is preferably a cell population containing as many chondrocytes (e.g., chondrocytes that produce type II collagen: Col2-positive chondrocytes) having functionality useful for exerting a therapeutic or prophylactic effect by transplantation as possible, and also containing some cartilage-derived Tie2-positive cells having a residual potential to produce such chondrocytes.

### - Cell Population Involving Stage Other Than Differentiation Culture Stage

In the present invention, a stage other than the differentiation culture stage may be optionally performed. In this case, the same matters as for the pre-differentiation culture cell population or the post-differentiation culture cell population described above can be said to apply to the cell population to be subjected to the step included in the stage and the cell population obtained by the step included in the stage. For example, it is possible to use, as the cell population to be subjected to the amplification culture step, a cell population like the pre-differentiation culture cell population described above. For example, the cell population obtained by the amplification culture step may be divided into a suitable number of cells in accordance with an embodiment in the differentiation culture stage (e.g., the type and size of cultureware) and then used.

### <Culture Medium>

The culture medium used in the culture method of the present invention or the preparation method of the present invention (each culturing step) may be any culture medium as long as it is suitable for culturing cartilage-derived Tie2-positive cells and chondrocytes differentiated therefrom. A suitable basal culture medium and a suitable additive component(s) may be selected in consideration of, for instance, the purpose of the culture method or the culturing step. Regarding the culture medium for the culture method of the present invention or the differentiation culture step of the present invention, an additive component(s) suitable for inducing differentiation from cartilage-derived Tie2-positive cells into chondrocytes, which is a main object, may be selected. They may be used together with the Tie2 expression enhancer other than growth factors. The culture medium in the additional differentiation culture step or the step (e.g., the amplification culture step) included in a stage other than the differentiation culture stage, which step is optionally performed in the present invention, may also be prepared by selecting an appropriate basal medium and an appropriate additive component(s) according to the purpose of the step.

In a representative embodiment of the present invention, cartilage-derived Tie2-positive cells and/or chondrocytes differentiated therefrom (mixed cell population thereof) may be cultured. For this purpose, the culture medium for each step at the differentiation culture stage or other culture stage, for example, the culture medium for the differentiation culture step, the additional differentiation culture step, and/or the amplification culture step of the present invention may each be prepared, for example, by using appropriate amounts of the following basal culture medium, additive component(s), growth factor(s), and other component(s).

Examples of the basal culture medium for plane culture include DMEM (Dulbecco's Modified Eagle Medium, without or with glucose), αMEM (a-modified Eagle's Minimum Essential Medium), Ham's F-10 medium, Ham's F-12 medium, or a mixture thereof (e.g., a culture medium prepared by mixing DMEM and F-10 or F-12 medium). In addition, examples of the basal culture medium for suspension culture (three-dimensional culture) include a methylcellulose medium (e.g., trade name "MethoCult", STEMCELL Technologies, Inc.).

Examples of the component(s) added to the basal culture medium include FBS (fetal bovine serum), BSA (bovine serum albumin), L-ascorbic acid (e.g., as L-ascorbic acid magnesium phosphate), selenious acid (e.g., as insulin-transferrin-sodium selenite (ITS: Insulin-Transferrin-Selenium)), and/or 2-mercaptoethanol. If necessary, antibiotics such as penicillin and streptomycin and other component(s) may be further added to the culture medium.

Examples of the growth factor(s) include FGF (fibroblast growth factor), EGF (epidermal growth factor), and/or Ang-1 (Angiopoietin-1). In an embodiment of the present invention, it is preferable to use at least FGF as the growth factor to be added to the culture medium, it is more preferable to use both FGF and EGF, and it is still more preferable to optionally use Ang-1 in addition to FGF and EGF.

Examples of FGF include bFGF (basic fibroblast growth factor; sometimes referred to as FGF-2). The concentration of FGF in the culture medium may be usually in the range of 1 to 50 ng/mL and preferably in the range of 5 to 15 ng/mL, for example, about 10 ng/mL.

Ang-1 is preferably added to a serum-free culture medium. Ang-1 is preferably solubilized in water (soluble Ang-1, recombinant Ang-1). The concentration of Ang-1 (preferably soluble Ang-1) in the culture medium may be usually in the range of 100 to 1000 ng/mL, for example, about 500 ng/mL.

Note that the above growth factors such as FGF, EGF, and Ang-1 are "growth factors having a Tie2 expression-enhancing effect", and can also be interpreted to correspond to a "Tie2 expression enhancer" in a broad sense, but the way of handling these growth factors in the present invention is as separately described herein. Specifically, the culture medium for the differentiation culture step in the present invention contains at least "a Tie2 expression enhancer other than growth factors" (e.g., an animal/plant-derived extract, preferably a plant-derived extract), and may further contain a "growth factor(s) having an action of enhancing Tie2 expression" such as FGF, EGF, and/or Ang-1. On the other hand, the culture medium for the additional differentiation culture step is free of any "Tie2 expression enhancer other than growth factors" (e.g., an animal/plant-derived extract), but may contain (preferably contain) "a growth factor(s) having an action of enhancing Tie2 expression" such as FGF, EGF, and/or Ang-1.

In an embodiment of the present invention, it is possible to use, as the culture medium for the differentiation culture step in the present invention, a culture medium prepared by adding 2-mercaptoethanol, selenious acid, ascorbic acid, and 30% BSA to a mixed culture medium of DMEM and F-10 medium, and further adding 30% FBS immediately before use.

In an embodiment of the present invention, the additional differentiation culture step is performed by suspension culture (three-dimensional culture), and a methylcellulose medium can be used as a culture medium for such an additional differentiation culture step.

In an embodiment of the present invention, it is possible to use, as the culture medium for the amplification culture step, αMEM to which 20% FBS is added and a growth factor(s) such as FGF is further optionally added.

### <Tie2 Expression Enhancer>

In the culture method of the present invention, at least one kind of "Tie2 expression enhancer" other than growth factors having a Tie2 expression-enhancing effect is added to the culture medium. When the culture method of the present invention is carried out in the step at the differentiation culture stage (as the differentiation culture step in the present invention), addition of the Tie2 expression enhancer acauses an effect of increasing the number or percentage of cells differentiated into functional chondrocytes while keeping the cartilage-derived Tie2-positive cells in a juvenile state (keeping a proliferation capability and a potential to differentiate into at least chondrocytes). Any one kind of the Tie2 expression enhancer may be used, or two or more kinds thereof may be used in combination. The enhancer may be added to the culture medium in an amount by which the Tie2 activity and effect as described above are elicited.

Examples of the "growth factor(s) having a Tie2 expression-enhancing effect" include Angiopoietin-1 (Ang-1) and/or FGF2 (bFGF). In the culture method of the present invention, at least one kind of "Tie2 expression enhancer" other than growth factors having a Tie2 expression-enhancing effect is added. However, a growth factor(s) having a Tie2 expression-enhancing effect may be optionally used in combination. When the culture method of the present invention is carried out in the step at the differentiation culture stage (as the differentiation culture step in the present invention), a growth factor having a Tie2 expression-enhancing effect and another Tie2 expression enhancer, for example, an animal/plant-derived extract as described below, preferably a plant-derived extract may be used in combination to elicit a synergistic effect.

The Tie2 expression enhancer other than growth factors can be used is each animal/plant-derived extract known as a "Tie2 activator" in the art. Examples of such an animal/plant-derived extract include an extract from autumn olive, Indian lettuce, Indian date, turmeric, *Engelhardia roxburghiana*, *Polygonatum rhizome,* psyllium, saltwort, olive fruit, oysters, camomile, Chinese quince, trichosanthes seed, Morinda officinalis root, chrysanthemum, *Polygonatum odoratum,* quillaia, ginkgo, harlequin glory bower, Chinese matrimony vine, sawtooth oak, Pink porcelain lily, Chinese ginseng, *Quercus serrata,* hawthorn, *Pellionia minima*, guajava, Siberian ginseng, star apple, star fruit, *Gleditsia officinalis Hemsl*., jujube, cinnamon, wild rocambole, lotus, *Colocasia gigantea*, *Kalopanax pictus,* long pepper, butcher bloom, mango ginger, bladdernut, *Stauntonia hexaphylla*, *Hemerocallis fulva var. kwanso,* bayberry, Japanese clethra, or rooibos (see Patent Documents 4 to 10). In addition, a component(s) contained in such an extract is, for example, a compound(s) such as ursolic acid, colosolic acid, 3-O-galloylprocyanidin B-1, linolenic acid, 13-hydroxy-9Z,11E,15E-octadecatrienoic acid, procyanidin B-2, epicatechin-(4β-6)-epicatechin(4β-8)-epicatechin, procyanidin C-1, astragaloside VIII, soya saponin I, 3'-O-methyl gallocatechin, pipernonaline, syringaresinol, 2-methoxycinnamaldehyde, eleutheroside E, eleutheroside E1, sesamin, eudesmin, sylvatesmin, pinoresinol, yangambin, forsythinol, coumarin. Each compound (see Patent Documents 7 and 12 to 14) can also be used as a Tie2 expression enhancer other than growth factors. For each extract or component, for instance, the dosage at which the Tie2 expression-enhancing effect is recognized, the portion (material) of plant/animal and the extraction process suitable for preparation, and/or the procedure for purifying a specific component(s) may also be set based on methods conventionally known to those skilled in the art, if appropriate.

From an industrial point of view, it is advantageous to use, as the Tie2 expression enhancer in the differentiation culture step in the present invention, one or two or more kinds selected from the above animal/plant extracts, preferably one or two or more kinds selected from the above plant-derived extracts, which are less expensive than growth factors such as Ang-1 and FGF2, preferably have a better Tie2 expression-enhancing effect than those growth factors, and more preferably exhibit a synergistic effect when used in combination with those growth factors.

### - Extract Derived from Plant of Genus Cinnamomum

In a preferred embodiment of the present invention, an extract derived from a plant of the genus *Cinnamomum* may be used as the Tie2 expression enhancer. The genus *Cinnamomum* includes 300 or more species such as *Cinnamomumcassia Blume,* C. *camphora,* C. *daphnoides,* C. *doederleinii*, C. *japonicum*, C. *pseudo-pedunculatum*, *C. sieboldii*, *C. verum,* or *C. zeylanicum*. For example, an extract of young twigs of *Cinnamomum cassia* or a bark of *Cinnamomum cassia,* or a product manufactured and sold as cinnamon powder obtained by processing them into powder can be used as an extract derived from a plant of the genus *Cinnamomum* in the present invention.

The extract derived from a plant of the genus *Cinnamomum* may be obtained by a conventional procedure, and can be prepared, for example, by immersing or heating and refluxing, a plant body (e.g., cinnamon powder) as a raw material at normal temperature or by heating together with an extraction solvent, and then recovering the supernatant, or by filtering a filtrate and optionally concentrating the filtrate. The extraction solvent used may be a solvent usually used for extraction. Examples include an aqueous solvent such as water, saline, phosphate buffer, or borate buffer. Alternatively, examples include an organic solvent such as an alcohol compound (e.g., ethanol, propylene glycol, 1,3-butylene glycol, glycerin), an aqueous alcohol compound, chloroform, dichloroethane, carbon tetrachloride, acetone, ethyl acetate, or hexane. They may be used singly or may be used in combination. Preferably, water is used as the solvent. The extract obtained by extraction with the above solvent may be used as it is in the form of an extraction liquid. However, from the viewpoint of convenience, the extract may be solidified (powderization) by, for instance, drying or lyophilization, stored, optionally diluted or re-dissolved (re-dispersed) with a suitable solvent upon use, further optionally subjected to treatment such as filtration, and then used. The extract derived from a plant of the genus *Cinnamomum* may be an extract (purified product) obtained by removing impurities by, for instance, an adsorption process using an ion exchange resin (e.g., a porous polymer such as Amberlite XAD-2), if necessary.

The concentration of the extract derived from a plant of the genus *Cinnamomum* in the culture medium can be adjusted, if appropriate, depending on the properties of the extract to be used, or in consideration of, for instance, the degree of effects as a Tie2 expression enhancer. For example, an extract obtained by extracting 10 mg of cinnamon powder with 1 mL of water (distilled water) may be used as the extract derived from a plant of the genus *Cinnamomum.* In this case, the above extract may be added in an amount of about 0.1 to 10 v/v%, for example, about 2 v/v% based on the culture medium. Even if the embodiment of extraction and addition is changed, the active ingredient as the Tie2 expression enhancer may be made comparable to that in the embodiment of extraction and addition described above.

Note that the extract derived from a plant of the genus *Cinnamomum* as obtained as described above contains various compounds. Each compound that can exert the effects of the present invention is not particularly limited. However, for example, syringaresinol (a kind of compound classified into furofuran-type lignan compounds) may be one of compounds having a Tie2 expression-enhancing effect in the present invention.

### <Culture Period and Other Conditions>

Basically, the period and other conditions (e.g., pH, CO₂ level, O₂ level) of each culturing step included in the culture method of the present invention or the preparation method of the present invention (at each culture stage) may be adjusted, if appropriate, so as to obtain a cell population having a desired cell composition (type and number/ratio) according to the purpose of (the culture stage including) the culturing step. The pH may be weakly alkaline (e.g., about 7.15). The CO₂ level may be, for example, about 5%. The O₂ level may be 5% or less (e.g., about 2%). During the period of the culture method of the present invention or each culturing step (stage), the culture medium may be optionally changed, if appropriate, with a fresh one every predetermined days. Also, the culture medium may be modified or the atmosphere may be changed by adding a component, or increasing or decreasing the concentration of the component or the pH, after a predetermined number of days has passed.

The period of the culturing step at the differentiation culture stage in the present invention (if the differentiation culture stage includes a plurality of culturing steps or, for example, if the stage includes the differentiation culture step and the additional differentiation culture step in the present invention, the culture period of each step) is usually about 1 to 3 weeks. For example, the differentiation culture step in the present invention may be performed for about 1 week, and the additional differentiation culture step may be performed for about 2 weeks. When the desired post-differentiation culture cell population is obtained, the differentiation culture stage may be terminated.

In the present invention, the period of the culturing step at the amplification culture stage optionally performed (if the amplification culture stage includes a plurality of culturing steps, the culture period of each step) is usually about 1 to 3 weeks, and may be, for example, about 1 week. When the desired post-amplification-culture cell population is obtained, the amplification culture stage may be terminated.

### <Cultureware>

Basically, cultureware, a culturing device, and others used in each culturing step included in the culture method of the present invention or the preparation method of the present invention (at each culture stage) may be selected, if appropriate, according to the purpose of the culture method or (the culture stage included in) the culturing step so as to obtain a cell population having a desired cell composition (type and number/ratio).

The cultureware used may be cultureware having a common shape, such as a flask, a dish, a plate, or a bag, and may have a well(s) capable of accommodating cells. The cultureware used may be cultureware made of a common material such as glass, plastic, or resin. The size (area or volume) of cultureware and, if the cultureware includes wells, the size (aperture and depth) and number of the wells, for instance, may also be selected, if appropriate.

For example, the culture method of the present invention, the differentiation culture step in the present invention, or the amplification culture step optionally performed in the present invention is typically performed by plane culture in which cells are attached to a culture surface of cultureware. Such plane culture is preferably performed using cultureware coated with a given coating agent, but may be performed using other known means for plane culture, such as using cultureware not coated with such a coating agent.

The additional differentiation culture step optionally performed in the present invention is typically performed by suspension culture in which cells are suspended in a culture medium. Such suspension culture can be performed, for example, by using a viscous methylcellulose medium as a culture medium, or cultureware having a low-adherent treated culture surface (coated with a substance for preventing cell adhesion), by optionally shaking, with, for instance, a culture shaker, .

### - Coating Agent

In the culture method of the present invention and the differentiation culture step in the present invention, it is preferable to use cultureware (herein sometimes simply referred to as "coated cultureware") having a treatment culture surface to be coated with a coating agent containing an extracellular matrix (ECM) or another biologically relevant molecule. Note that the amplification culture step may be likewise performed using coated cultureware.

Examples of the ECM contained in the coating agent in the present invention include various known ECMs such as collagen (e.g., type I, type II, type IV collagen) or gelatin as a heat-treated product thereof, chondroitin sulfate A, fibronectin, gelatin, laminin, thrombospondin, vitronectin, or proteoglycan (e.g., aggrecan, heparin sulfate proteoglycan). Examples of the biologically relevant molecule other than ECM include a polyamino acid such as polylysine (poly-L-lysine or poly-D-lysine). Examples of another coating agent include polyglycolic acid, PLGA (a polylactic acid-glycolic acid copolymer), polyhydroxyalkanoic acid (PHA), poly-ε-caprolactone, polyorthoester, polyacid anhydride, polyphosphazene, polydimethylsiloxane, polyurethane, polytetrafluoroethylene, polyethylene, polysulfone, polymethyl methacrylate, poly-2 hydroxyethyl methacrylate, polyamide, polypropylene, polyvinyl chloride, polystyrene, polyvinylpyrrolidone, or polyornithine. The coating agent for cell attachment treatment may contain any one of the above-mentioned substances, or may contain two or more kinds thereof. In one preferred embodiment of the present invention, the coating agent contains a polyamino acid such as polylysine (e.g. poly-L-lysine).

### -Composition for Cell Therapy-

The composition for cell therapy according to the present invention may contain a cell population obtained by the culturing method or the preparation method of the present invention as described above, that is, chondrocytes (in particular, functional chondrocytes such as Col2-positive cells) differentiated from cartilage-derived Tie2-positive cells, preferably contains a cell population including a certain amount of cartilage-derived Tie2-positive cells, and may optionally contain an additional pharmaceutically acceptable component(s).

In an exemplary embodiment of the present invention, the composition for cell therapy is for treating or preventing an injury, symptom, or disease in cartilage. The composition for cell therapy in this embodiment is applied to an injury, symptom or disease in cartilage that can be prevented or treated by administering the composition. Examples thereof include cartilage injury, osteochondral injury, meniscus injury, arthritis, or arthropathy.

The dosage form of the composition for cell therapy in the present invention may be any form as long as the cell population can be transplanted or delivered to a target site (e.g., a cartilage-containing tissue such as a joint). Examples include an injection, preferably an injection for topical administration to cartilage or the vicinity thereof, or an injection for vascular administration allowing for targeting.

Examples of the pharmaceutically acceptable component(s) include water for injection or physiological saline used in the case of preparation as an injection, culture liquid for the cell population, other suitable solvent/dispersion medium, and/or other additive(s).

The composition for cell therapy according to the present invention may be administered in an amount effective in eliciting a desired therapeutic or prophylactic effect. While the ingredient(s) of the composition for cell therapy, the dosage form, the administration subject, the administration route, and other embodiments are considered, such an effective amount may be adjusted, if appropriate, by, for instance, the dose per administration, the number of administrations, and/or the dosing interval (the number of administrations within a certain period). The composition for cell therapy according to the present invention can be administered to humans or non-human animals.

### [Examples]

In the following Examples, commercially available knee joint chondrocytes (2nd passage of cell population that was derived from normal adult knee joint cartilage) (hereinafter, referred to as "NHAC2") were used as the "cell population containing cartilage-derived Tie2-positive cells " in the present invention.

As a culture medium for the culturing step at the "amplification culture stage" in Examples (hereinafter, referred to as a "culture medium for amplification culture step"), a culture medium prepared by adding 20% FBS to αMEM immediately before use was used.

A culture medium used for the first culturing step at the "differentiation culture stage" in the Examples (hereinafter, referred to as a "culture medium for differentiation culture first step") was a culture medium prepared by mixing 60 mL of DMEM (no glucose) and 40 mL of F10, by adding 1 pL of 2-mercaptoethanol, 6 pL of selenious acid (0.01%), 1.5 mL of ascorbic acid (5 mg/mL), and 5 mL of 30% BSA, and by further adding FBS in an amount of 30% to the culture medium and "2% cinnamon" or "10 ng/mL bFGF" immediately before use. The "2% cinnamon" means that "10 mg of commercially available cinnamon powder was suspended in 1 mL of distilled water and extracted at 37°C overnight to obtain an extract (cinnamon extract)" and this extract was added in an amount of 2% to the culture medium.

As a culture medium for the second culturing step at the "differentiation culture stage" in Examples (hereinafter, referred to as a "culture medium for differentiation culture second step), a methylcellulose medium (trade name "MethoCult H4230", STEMCELL Technologies, Inc.; free of growth factors) was used.

### [Example 1]

NHAC2 was dispersed in the culture medium for amplification culture step, and plane culture was started in one well of a 6-well culture dish coated with poly-L-lysine (PLL) (day 0).

After 7 days (day 7), the cell population after the amplification culture step was collected and dispersed in a culture medium for differentiation culture first step (containing either "2% cinnamon" or "10 ng/mL bFGF"), and planar culture was started in one well of a 6-well culture dish coated with PLL.

After 7 days (day 14), the cell population after the differentiation culture first step was collected and dispersed in a culture medium for differentiation culture second step, and suspension culture was started in one well of a 6-well culture dish without coating treatment. After 14 days (day 28), the cell population after the differentiation culture second step was collected. The number of cells positive for expression of Tie2 on the cell surface was measured by flow cytometry method (FCM), and the percentage (Tie2-positive rate) of the number of Tie2-positive cells to the number of cells of the entire cell population was calculated. In the FCM procedure, a fluorescently labeled agent, which is a complex of an anti-human Tie2 antibody and a fluorescent die Allophicocyanin (Anti-Tie-2, Human, Mouse-Mono (87315); Allophicocyanin, Cat#: FAB 3131A; R&D Inc.), was used. The above culture and measurement were triplicate.

The results of the Tie2-positive rate (Mean ± SEM, n = 3) are shown in Fig. 1. The Tie2-positive rate in the obtained cell population was significantly higher in the case of adding "2% cinnamon" than in the case of adding "10 ng/mL bFGF" in the differentiation culture step (p < 0.05). The differentiation culture step in the present invention was found to exert an effect of enhancing Tie2 expression.

### [Example 2]

NHAC2 was separated into cells positive for Tie2 expression and cells negative for Tie2 expression by the FCM procedure. Each of Tie2-positive cells and Tie2-negative cells was cultured in substantially the same manner as in Example 1 (the amplification culture step, differentiation culture first step, and differentiation culture second step). For each of the Tie2-positive cells and the Tie2-negative cells, spherical (spheroid) colony forming units (CFU-S) and fibroblastic colony forming units (CFU-F) in the cell population after the differentiation culture second step were counted. The above culture and measurement were triplicate.

The results of each colony count per 1000 cells (Mean ± SEM, n = 3) are shown in Fig. 2. The spherical colony forming units (CFU-S) were significantly higher in the Tie2-positive cells than in the Tie2-negative cells (p < 0.05), indicating that the cartilage-derived Tie2-positive cells would have more strongly properties as spherical colony-forming stem/precursor cells.

### [Example 3]

Spherical (spheroid) colony forming units (CFU-S) and fibroblastic colony forming units (CFU-F) in the cell population after the differentiation culture second step when cultured in substantially the same manner as in Example 1 (the amplification culture step, differentiation culture first step, and differentiation culture second step) were measured, and compared by the difference of the component added to the culture medium in the differentiation culture first step (between "2% cinnamon " and "10 ng/mL bFGF"). The above culture and measurement were triplicate.

The results of each colony count per 1000 cells (Mean ± SEM, n = 3) are shown in Fig. 3[a]. In addition, an optical micrograph of a colony formed when "2% cinnamon" was added is shown in Fig. 3[b]. The spherical colony forming units (CFU-S) were significantly higher in the case of adding "2% cinnamon" than in the case of adding "10 ng/mL bFGF" (p < 0.05). The above results have demonstrated that use of the "Tie2 expression enhancer other than growth factors" (cinnamon as an example thereof) in the present invention causes an increase in the Tie2-positive rate of the cell population or promotes the expression thereof in the Tie2 positive cells. This indicates that the cell population containing cells which have more strongly properties as spherical colony-forming stem/precursor cells could be obtained thereby.

### [Example 4]

Cells were cultured in substantially the same manner as in Example 1 (the amplification culture step, differentiation culture first step, and differentiation culture second step). The cell population after the differentiation culture second step was collected. The number of cells positive for each expression of proteoglycan, type I collagen, or type II collagen on the cell surface was measured by flow cytometry (FCM), and the percentage (positive rate) of the cells to the number of cells containing the entire cell population was calculated.

The results of the positive rate (Mean ± SEM, n = 3) of each of proteoglycan (PG), type I collagen (Type 1 col), and type II collagen (Type 2 col) are shown in Fig. 4. The type II collagen positive rate in the obtained cell population was significantly higher in the case of adding "2% cinnamon" than in the case of adding "10 ng/mL bFGF" in the differentiation culture first step (p < 0.05). This has demonstrated that by performing the differentiation culture step (differentiation culture first step) according to the culture method of the present invention, a cell population rich in functional chondrocytes positive for expression of type II collagen can be prepared.

## Claims

1. A method of culturing a cell population containing cartilage-derived cells which are positive for expression of Tie2 (tyrosine kinase with Ig and EGF homology domain-2) (hereinafter referred to as "cartilage-derived Tie2-positive cells"), the method comprising:
culturing a cell population containing cartilage-derived Tie2-positive cells in a culture medium containing at least one kind of plant-derived extract having a Tie2 expression-enhancing effect.

2. The culture method according to claim 1, wherein the plant is a plant of the genus *Cinnamomum.*

3. The culture method according to claim 1 or 2, wherein the culturing is performed in cultureware having a culture surface coated with a coating agent containing an extracellular matrix and/or a polyamino acid.

4. The culture method according to claim 3, wherein the extracellular matrix is type IV collagen and/or fibronectin.

5. The culture method according to claim 3 or 4, wherein the polyamino acid is polylysine.

6. A method of preparing a cell population containing chondrocytes differentiated from cartilage-derived Tie2-positive cells, the method comprising:
a culture stage (hereinafter, referred to as an "differentiation culture stage") comprising a step of performing the culture method according to any one of claims 1 to 5 in order to enhance expression of Tie2 on cartilage-derived Tie2-positive cells in a cell population and induce differentiation into chondrocytes.

7. The preparation method according to claim 6, wherein the chondrocytes comprise cells positive for expression of Col2 (type II collagen).

8. The preparation method according to claim 6 or 7, wherein the differentiation culture stage further comprises a step of culturing the cell population containing the cartilage-derived Tie2-positive cells in a culture medium containing at least one growth factor selected from the group consisting of FGF (fibroblast growth factor), EGF (epidermal growth factor) and Ang-1 (angiopoietin-1).

9. The preparation method according to any one of claims 6 to 8, further comprising, before the differentiation culture stage, a culturing stage of amplifying the cell population containing the cartilage-derived Tie2-positive cells (hereinafter, referred to as an "amplification culture stage").

10. The preparation method according to any one of claims 6 to 9, wherein a cell population in which the cartilage-derived Tie2-positive cells also remain is obtained through the differentiation culture stage.

11. A cell population obtained by the culture method according to any one of claims 1 to 5.

12. A culture comprising a culture medium in the culture method according to any one of claims 1 to 5 and a cell population to be subjected to the culture method, being cultured, or produced.

13. A cell population obtained through the differentiation culture stage in the preparation method according to any one of claims 6 to 10.

14. A culture comprising a culture medium for the differentiation culture stage and a cell population to be subjected to the differentiation culture stage, being cultured, or produced, in the preparation method according to any one of claims 6 to 10.

15. A composition for cell therapy, comprising the cell population according to claim 10 or 13.

16. The composition for cell therapy according to claim 15, which is for treatment or prevention of a disorder, symptom, or disease in cartilage.
